# EUROPEAN PATENT SPECIFICATION

(11) **EP 2 614 047 B1**
(45) Date of publication and mention of the grant of the patent: **26.10.2016**
(21) Application number: 11752493.4
(22) Date of filing: 05.09.2011
(51) Int. Cl.: C07C 251/20, C07C 255/34, H01L 51/42, C07C 49/665, C07D 213/24, C07D 333/06

(54) **COMPOUNDS FOR ORGANIC PHOTOVOLTAIC DEVICES**
VERBINDUNGEN FÜR ORGANISCHE PV-ELEMENTE
COMPOSÉS POUR DISPOSITIFS PHOTOVOLTAÏQUES ORGANIQUES

(30) Priority: 10.09.2010 EP 10400044
(43) Date of publication of application: 17.07.2013
(73) Proprietor: Novaled AG, 01307 Dresden (DE)
(72) Inventor: FADHEL, Omrane, 01099 Dresden (DE); DOROK, Sascha, 01099 Dresden (DE); HARTMANN, Horst, 01326 Dresden (DE); PRETSCH, Ramona, 01097 Dresden (DE)
(74) Representative: Bittner, Thomas L.
(86) International application number: PCT/EP2011/004468
(87) International publication number: WO 2012/031735

(56) References cited:
- US-A- 5 683 833
- US-A1- 2003 008 174
- US-A1- 2007 289 625
- DATABASE CA [Online] CHEMICAL ABSTRACTS SERVICE, COLUMBUS, OHIO, US; 2008, OKADA, HIDEKI ET AL: "Electrophotographic photoconductor containing fluorenone derivative electron-transporting agent", XP002667095, retrieved from STN Database accession no. 2008:1338042 & DATABASE WPI Week 200881 Thomson Scientific, London, GB; AN 2008-N93197 & JP 2008 268804 A (MITA IND CO LTD) 6 November 2008 (2008-11-06)
- DATABASE CA [Online] CHEMICAL ABSTRACTS SERVICE, COLUMBUS, OHIO, US; ALKORTA, IBON ET AL: "Theoretical study of racemization in chiral alkenylidene truxenes", XP002668234, retrieved from STN Database accession no. 2008:759066 & ALKORTA, IBON ET AL: "Theoretical study of racemization in chiral alkenylidene truxenes", JOURNAL OF PHYSICAL ORGANIC CHEMISTRY , 21(5), 381-386 CODEN: JPOCEE; ISSN: 0894-3230, 2008,

## Description

### Technical Field

This invention is related to organic photovoltaic (OPV) devices, also known as organic solar cells.

The solar light is one of the most attractive forms of renewable energy, because it is available in relatively large power density, and is easily convertible into other forms of energy such as electrical, thermal, etc.

Organic solar cells offer a big promise for the efficient and large scale conversion of light into electricity. The production of organic solar cells is less material demanding than the production of inorganic crystalline solar cells. The production also consumes considerably less energy than the production of any other inorganic solar cell.

Efficiency of organic solar cells has been improving steadily. In 2008 a certified power conversion efficiency value of 5% was reached, and in 2010 the psychological barrier of 8% was broken, aligning the efficiency of the organic solar cells to typical values of amorphous Si devices.

### Background art

OPV devices have the most different devices architectures. Typically they comprise at least one organic semiconducting layer between two electrodes. That organic layer can be a blend of a donor and an acceptor such as P3HT (poly3-hexyl-thiophene) and PCBM (phenyl Cn Butyric Acid Methyl Ester). Such simple device structures only achieve reasonably efficiencies if interfacial injection layers are used to facilitate charge carrier injection/extraction (Liao et al., Appl. Phys. Lett., 2008. 92: p. 173303). Other organic solar cells have multi-layer structures, sometimes even hybrid polymer and small molecule structures. Also tandem or multi-unit stacks are known (Ameri, et al., Energy & Env. Science, 2009. 2: p. 347). Multi-layer devices can be easier optimized since different layers can comprise different materials which are suitable for different functions. Typical functional layers are transport layers, optically active layers, injection layers, etc.

Optically active materials are materials with a high absorption coefficient, for at least a certain wavelength range of the solar spectra, which materials convert absorbed photons into excitons which excitons contribute to the photocurrent. The optically active materials are typically used in a donor-acceptor heterojunction, where at least one of the donor or acceptor is the light absorbing material. The interface of the donor-acceptor heterojunction is responsible for separating the generated excitons into charge carriers. The heterojunction can be a bulk-heterojunction (a blend), or a flat (also called planar) heterojunction, additional layers can also be provided (Hong et al, J. Appl. Phys., 2009. 106: p. 064511).

The loss by recombination must be minimized for high efficiency OPV devices. Therefore, the materials in the heterojunction must have high charge carrier mobilities and high exciton diffusion lengths. The excitons have to be separated at the heterointerface and the charge carriers have to leave the optically active region before any recombination takes place. For that reasons, only few organic materials are suitable to be used in the heterojunction. For instance, currently, there are no known materials which can compete with the fullerenes (C60, C70, PCBM, and so on) as acceptor in OPV devices.

Transport materials are required to be transparent, at least in the wavelengths wherein the device is active, and have good semiconducting properties. These semiconducting properties are intrinsic, such as energy levels or mobility, or extrinsic such as charge carrier density. The charge carrier mobility can also be extrinsically influenced, for instance, by doping the material with an electrical dopant.

JP 2008-268804 A discloses a photoreceptor comprising a conductive support having a photosensitive layer containing an electron-transporting agent having a condensed aromatic structure, a charge-generating agent and a binder.

Alkorta et al., Journal of Physical Organic Chemistry, 2008, 21(5), 381-386 investigates the racemization process of chiral truxene derivatives using DFT-based methods.

US 2003/0008174 A1 discloses an organic luminescent device which is constituted by a pair of an anode and a cathode, and at least one organic layer disposed between the anode and the cathode. The organic layer includes a layer of a fused polynuclear compound.

US 2007/0289625 A1 is related to monomeric, oligomeric or polymeric compound for use in an optoelectronic device, such as a photovoltaic cell, a field-effect transistor or an electrochemical sensor.

US 5,683,833 discloses the use of organic materials having a specific conductivity of less than 10⁻²S/cm and a non-ionic charge carrier mobility greater than 10⁻⁴ cm²/Vs as charge transport medium, with the proviso that an increase in the charge carrier concentration by a factor of 10 or more is not caused in this organic material by light absorption, and corresponding electrochemical cells.

Although in steady development, the choice of materials for OPV is still very limited, especially for optically active materials and for electron transport materials. Some highly efficient device structures employ TiO as electron transport and optical spacer with the disadvantage of being difficult to deposit (Simon et al., Int. J. of Mat. & Prod. Tech., 2009. 34: p. 469). Other devices use Fullerene C60 as ETL which is not transparent enough for functioning as an optical spacer. Other materials such as NTCDA (1,4,5,8-naphthalene-tetracarboxylic dianhydride), although transparent and with good semiconducting properties, are not morphologically stable and crystallize even at room temperature.

Almost no organic electron transport material is available with suitable semiconducting, chemical, and thermal properties.

### Technical Problem

It is the objective of the present invention to provide a new organic semiconductor material for use in organic solar cells. The material is preferentially used in at least one of an optically active layer, and an electron transport layer, more preferentially in an electron transport layer.

### Solution of the problem

This object is achieved by the subject-matter according to the independent claims. Preferred embodiments result from the sub-claims.

Described herein are new compounds according to Formula (I). The object is achieved by the independent claim 1, and by the invented preferred uses and devices of the dependent claims. This object is in particular achieved by an organic solar cell comprising at least one compound according to the following formula (I): 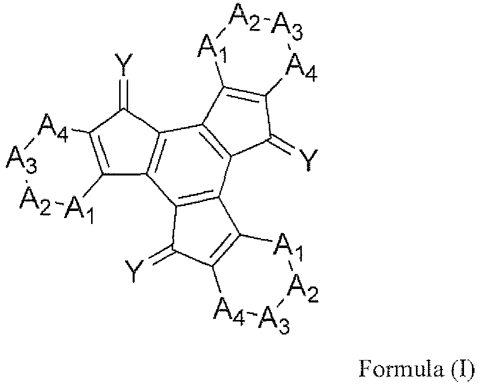 wherein
Y is selected from N-R₁ or R₁-C-R₂;
R₁ and R₂ are independently selected from CN, aryl, alkyl, NO₂, halogen, heteroaryl, CF₃, wherein aryl can be substituted to form perfluorophenyl and/or flurophenyl,
A₁,A₂,A₃,A₄ are independently selected from C, CH, N, CR, or at least one of A₁-A₂, A₂-A₃, A₃-A₄ is part of an additional condensed aromatic ring, the additional aromatic condensed ring being preferably: phenyl, pyrazyl, thienyl, imidazolyl, heteroaryl;
R is an aromatic ring, preferably aryl as defined below, or the compound according to formula (I) is selected from a compound according to the following formulae (Ia) and (Ib) 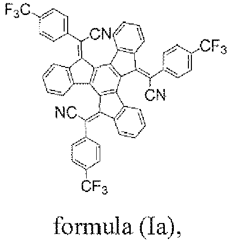 or

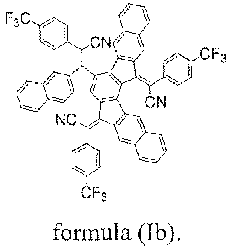

It is further preferred that R₁ is CN and R₂ is aryl or heteroaryl.

Aryl is preferably selected from: phenyl, naphthyl, anthracyl.

Heteroaryl is preferably C₅-C₂₀ heteroaryl, more preferably selected from pyridyl, thienyl, oxazyl.

The term "aryl" means an aromatic group containing only carbon in the aromatic ring or rings. An aryl group may contain 1 to 3 separate, fused, or pendant rings and from 6 to 20 ring atoms, without heteroatoms as ring members. Examples of aryl groups include, but are not limited to, phenyl, naphthyl, including 1-naphthyl and 2-naphthyl, perfluorophenyl, fluorophenyl, and biphenyl.

The object is further achieved by a compound

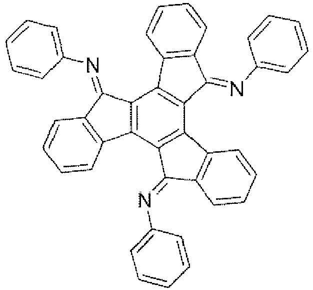

### Advantages of the compounds

A compound according to formula (I) is preferentially used in an electron transport layer in a solar cell. The compound is the main component of the electron transport layer. Preferentially at least one electron transport layer comprising the compound according to formula (I) is doped with an electrical dopant.

In an alternative embodiment, or in addition, the compound according to formula (I) is used in an exciton blocking layer as its main component. The exciton blocking layer is preferentially also an electron transport layer. The layer has a low enough LUMO to transport electrons between the acceptor and the cathode, and at the same time, it has a high HOMO-LUMO gap to block the excitons confining them into the optically active region, which means that the HOMO-LUMO gap is larger than the HOMO-LUMO gap of any immediately (in contact) adjacent material from the optically active region. This layer is preferentially electrically undoped.

In another alternative embodiment, or in addition, the compound according to formula (I) is used as a main component of an exciton-and-hole-blocking-layer. In this embodiment, the exciton- and-hole-blocking-layer has a low enough HOMO to block holes from an adjacent layers (mainly from the donor molecule of the bulk-heterojunction), and at the same time, it has a high HOMO-LUMO gap to block the excitons out of its layer confining them into the optically active region, which means that the HOMO-LUMO gap is larger than the HOMO-LUMO gap of any immediately (in contact) adjacent material from the optically active region.. This layer is preferentially electrically undoped.

In an alternative embodiment, or in addition, the compound according to formula (I) is as acceptor in a donor-acceptor heterojunction. In an aspect of this embodiment, the compound also harvest light which light is converted into charge and contributes to the photocurrent, preferentially, the contribution of the photocurrent at 0V is due to absorption of photons in the range of 350-500 nm. Preferentially the contribution is greater than 5%.

In an alternative embodiment, or in addition, the compound according to formula (I) is used as main component of an exciton-blocking and electron-transporting layer. In this embodiment, the exciton-blocking-and-electron-transporting-layer has low enough LUMO to accept the electron from a donor molecule in an adjacent layer, and at the same time, it has a high HOMO-LUMO gap to block the excitons out of its layer confining them into the optically active region, which means that the HOMO-LUMO gap is larger than the HOMO-LUMO gap of any immediately (in contact) adjacent material from the optically active region. This layer is preferentially electrically undoped.

The compound according to formula (I) is preferentially used as main component of a layer in combination with an adjacent layer, wherein the module of the difference of the LUMO of the layer and the adjacent layer is smaller 0.4 eV, more preferentially smaller than 0.2 eV (0.2 eV is about the width of the density of states of one material).

Preferentially the adjacent layer comprises as its main component a fullerene chosen from C₅₈, C₆₀, C₇₀, or a soluble derivative of it (e.g. PC₆₀BM).

In another aspect of the invention, an organic solar cell comprises a compound according to the Formula (I), in a layer adjacent to the donor-acceptor heterojunction, in an undoped form. In addition, the organic solar cell comprises an additional doped layer comprising a compound according to the Formula (I) between the layer adjacent to the donor-acceptor heterojunction and the cathode.

In another aspect of the invention, the solar cell is a polymer solar cell, comprising at least one semiconducting polymer in the at least one donor-acceptor heterojunction and comprising the compound according to formula (I) in at least one electron transport layer. Preferentially at least one electron transport layer is n-doped.

In a preferred aspect of the invention, the organic solar cell comprises a pi, ni, or pin structure, comprising a first *p, i, or n* layer each. *Here, p* denotes a p-doped hole transport *layer, n* denotes a n-doped electron transport layer, and i is an intrinsic photoactive layer. The transport layers have a greater HOMO-LUMO gap than the photoactive layer.

For all aspects of the invention, typical n-dopants are: tetrathianaphthacene, [Ru(terpy)2]⁰; rhodamine B; pyronin B chloride; acridine orange base; leuco crystal violet; 2,2'-diisopropyl-1,1',3,3'-tetramethyl-2,2',3,3',4,4',5,5',6,6',7,7'-dodecahydro-1H, 1'H-2,2-bibenzo[d]imidazole; 4,4',5,5' - tetracyclohexyl - 1,1',2,2',3,3' - hexamethyl - 2,2',3,3' - tetrahydro- 1H,1'H-2,2'-bisimidazole; 2,2'-diisopropyl - 4,4',5,5' - tetrakis(4-methoxyphenyl) - 1,1',3,3'-tetramethyl-2,2',3,3'-tetrahydro-1H,1'H-2,2'-bisimidazole; 2-isopropyl-1,3-dimethyl - 2,3,6,7 - tetrahydro-1H-5,8-dioxa-1,3-diaza-cyclopenta[b]-naphthene; bis-[1,3-dimethyl-2-isopropyl-1,2-dihydro - benzimidazolyl-(2)]; tetrakis (1,3,4,6,7,8 - hexahydro - 2H - pyrimido [1,2-a] pyrimidinato) di-tungsten(II); 2,2' - diisopropyl - 4,5 - bis(2-methoxyphenyl) - 4',5' - bis(4-methoxyphenyl) - 1,1',3,3' -tetramethyl-2,2',3,3'-tetrahydro-1H,1'H-2,2'-bisimidazole; 2,2'-diisopropyl-4,5-bis(2-methoxyphenyl) - 4',5' - bis(3-methoxyphenyl) - 1,1',3,3' - tetramethyl - 2,2',3,3' - tetrahydro - 1H, 1'H - 2,2' - bisimidazole (see for example, patent publications US 2005/0040390, US 2009/0212280, and US 2007/0252140).

### Brief description of the drawings

Fig. 1 is a simple diagram representing the stack of layers which forms a solar cell.
Fig. 2 is a simple diagram representing the layers of a solar cell comprising an ETL.

### Devices

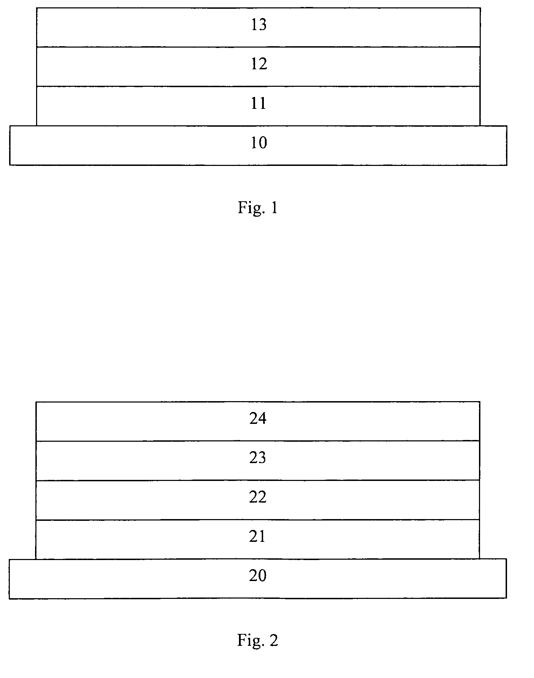

According to Fig. 1, an organic solar cell comprises at least a substrate (10), an anode (11), at least one organic optically active layer (12), and a cathode (13). The stack of layers can also be inverted, wherein layer (11) would be the cathode, and layer (12) would be the anode.

In one embodiment, the substrate (10) is a transparent substrate, such as a glass, or polymeric plate or web; the anode (11) is a transparent conducting oxide, such as ITO, FTO, AlZO; and the cathode (13) comprises aluminum or an aluminum alloy. In one embodiment the at least one organic optically active layer (12) comprises a blend of a thiophene containing polymer and a compound according to formula (I). Alternatively the at least one organic optically active layer (12) comprises a blend of a donor polymer, preferentially a thiophene containing polymer, and an acceptor, preferentially a fullerene or a soluble fullerene derivative; in this embodiment a layer containing the compound according to Formula (I) is formed between the at least one organic optically active layer (12) and the cathode (13). Optionally the layer structure is inverted.

In one embodiment the anode (11) is not transparent and mainly comprises Aluminum or an Aluminum alloy. The substrate (10) is not necessarily transparent. The cathode (13) comprises a transparent conducting oxide layer or a thin (thickness < 30 nm) transparent metal layer.

Still in connection to Fig. 1, in another embodiment, the substrate (10), the anode (11), and the cathode (13) are transparent. In this embodiment, the overall device is semi-transparent, because it does not have 100% absorption of the incident light for any wavelength in the visible range of wavelengths.

Note that multiple stacked devices (e.g. tandem devices) are also provided in this invention. In such devices at least one additional organic optically active layer is formed between the at least one organic optically (12) and the cathode (13). Additional organic or inorganic layers may be used to provide a suitable electronic connection and optical optimization of the layer position. Preferentially, at lest parts of these functions are provide by layers comprising a compound according to the formula (I).

Still in connection to Fig.1, surface treatment of the electrodes, buffer layers, and/or injection layers can be used to provide efficient charge carrier injection/extraction. Examples of surface treatments are acid, or plasma treatment of the electrode's surface. Example of injection layers are thin inorganic insulating layers (e.g. LiF) and thin electrical dopant layers.

Fig.2 shows a stack of layers representing an organic solar cell comprising at least: a substrate (20), an anode (21), at least one optically active layer (22), an organic electron transport layer (ETL) (23), and a cathode (24). The stack of layers can also be inverted. The ETL is formed between cathode and optically active layer.

In one embodiment, the organic electron transport layer comprises as its main component a compound according to the Formula (I). Preferentially this compound according to the Formula (I) is doped with an electrical dopant. The ETL (23) can have any thickness, its thickness is preferably smaller than 40 nm in the case that there is no additional optically active layer between the at least one optically active layer (22) and the cathode (24).

All embodiments as described in connection to fig. 1 can also be applied here, in connection to fig. 2.

All figures are simple representations of the layered structure of a solar cell. Some device features are not shown such as electrical connections, encapsulation, optical structures which are external to the electrodes, etc. At least one of the electrodes (anode and cathode) is transparent in the wavelength range in which the device is active.

In another embodiment the at least one optically active layer (22) is a donor-acceptor bulk heterojunction (blend of donor-acceptor). The donor is preferentially formed by a strong absorbing compound comprising a pyrrole or a thiophene group. The acceptor is preferentially a C₅₈, C₆₀, or C₇₀ fullerene or a soluble fullerene derivative. The ETL (23) comprises a compound according to the formula (I) as its main component. The ETL (23) is preferentially doped with an n-dopant. Or organic n-dopants are highly preferred due to their easier handling in production.

In another embodiment the at least one optically active layer (22) is a donor-acceptor bulk heterojunction (blend of donor-acceptor). The donor is preferentially formed by a strong absorbing compound comprising a pyrrole or a thiophene group. The acceptor is a compound according to Formula (I).

In one aspect of the invention, all organic layers are constituted from small molecules. Preferentially, these small molecules can be deposited by VTE (Vacuum Thermal evaporation).

In another aspect of the invention, at least one organic semiconducting layer comprises a polymer and at least one additional semiconducting layer comprises a compound according to Formula (I).

Another aspect of the invention is a layer comprising a compound of Formula (I) and an n-dopant. The invented compounds have a special advantage of forming very stable n-doped layers with a relatively high conductivity.

The conductivity can be measured by the so-called 2-point or 4-point-method. Here, contacts of a conductive material, such as gold or indium-tin-oxide, are disposed on a substrate. Then, the thin film to be examined is applied onto the substrate, so that the contacts are covered by the thin film. After applying a voltage to the contacts the current is measured. From the geometry of the contacts and the thickness of the sample the resistance and therefore the conductivity of the thin film material can be determined. The four point or two point method give the same conductivity values for doped layers since the doped layers grant a good ohmic contact.

The temperature stability can also be measured with that method in that the (undoped or doped) layer is heated stepwise, and after a waiting period the conductivity is measured. The maximum temperature, which can be applied to the layer without loosing the desired semiconducting properties, is then the temperature just before the conductivity breaks down. For example, a doped layer can be heated on the substrate with two electrodes, as disclosed above, in steps of 1°C, wherein after each step there is a waiting period of 10 seconds. Then the conductivity is measured. The conductivity changes with temperature and breaks down abruptly at a particular temperature. The temperature stability is therefore the temperature up to which the conductivity does not break down abruptly. The measurement is performed in vacuum.

The properties of the many different used materials can be described by the position of their highest occupied molecular orbital energy level (HOMO, synonym of ionization potential), and the lowest unoccupied molecular orbital energy level (LUMO, synonym of electron affinity).

Preferred compounds are:

| C-1 (comparative) | C-2 | C-3 | C-4 |
|---|---|---|---|
| 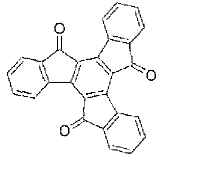 | 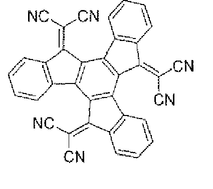 | 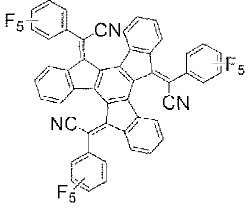 | 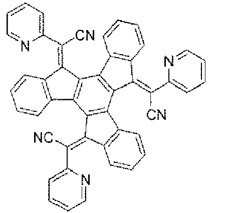 |

| C-5 | C-6 | C-7 | C-8 |
|---|---|---|---|
| 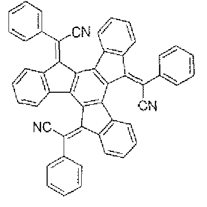 | 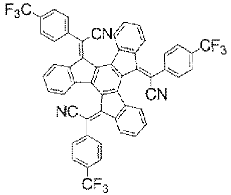 | 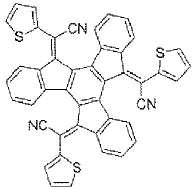 | 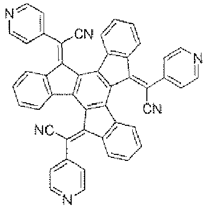 |

| C-9 | C-10 | C-11 | C-12 |
|---|---|---|---|
| 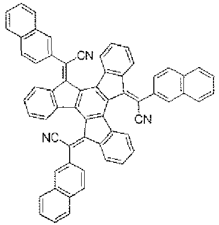 | 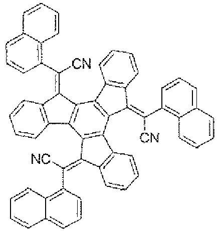 | 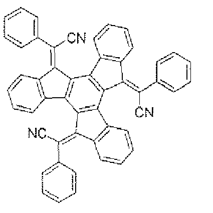 | 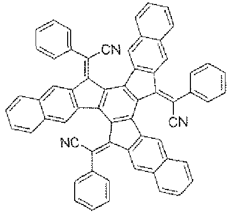 |

| C-13 | C-14 (comparative) | C-15 | C-16 |
|---|---|---|---|
| 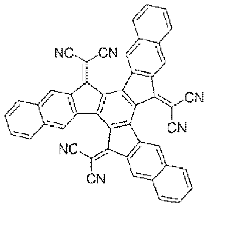 | 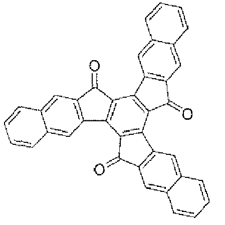 | 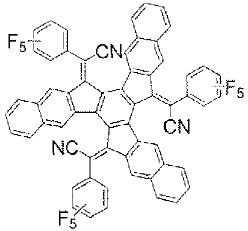 | 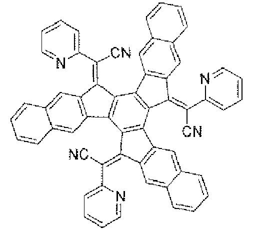 |

| C-17 | C-18 | C-19 | C-20 |
|---|---|---|---|
| 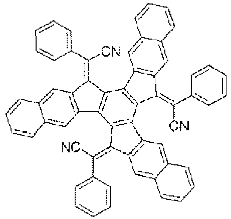 | 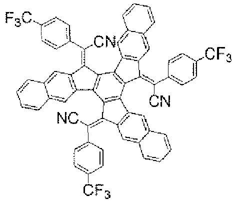 | 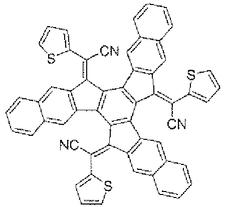 | 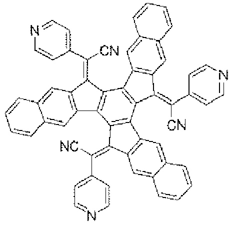 |

| C-21 | C-22 | C-23 | C-24 |
|---|---|---|---|
| 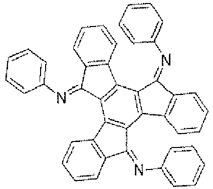 | 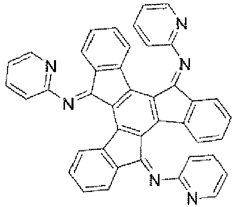 | 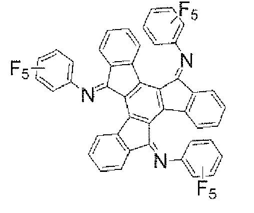 | 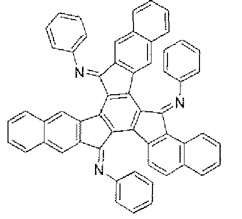 |

### Synthesis - General

The following schema describes the general synthesis for the compounds according to the invention. 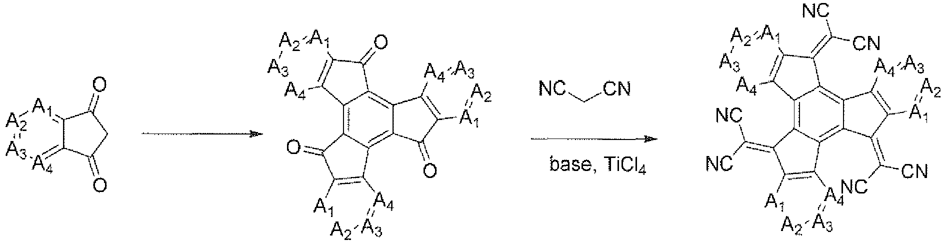 See ref: Sanguinet et al. Chem. Mater. 2006, 18, 4259-4269 and Jacob et al. Tetrahedron Letters 40 (1999) 8625-8628

### Synthesis - Examples

### Example C-2:

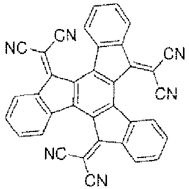

### Step 1:

First step: Synthesis of 5H-diindeno[1,2-a:1',2'-c]fluorene-5,10,15-trione (1). All manipulations were carried out in air, without any further purification of commercial solvents/chemicals.

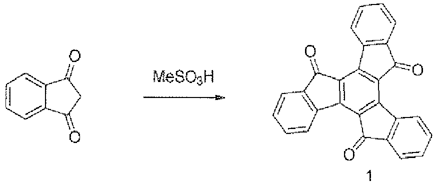

Truxenone. In a 100 mL round-bottom flask with a magnetic stir bar was added indan-1,3-dione (2.50 g, 17.0 mmol) and methanesulfonic acid (40 mL). The mixture was heated at 110 °C for 3 h. After being cooled to room temperature, the reaction mixture was dispersed in water (300 mL) and the crude product was filtered off. The product was dissolved in hot propylene carbonate (75 mL) and after being cooled was isolated by suction filtration.
Yield: 1.73 g (79%).
1H NMR (CDCl3): ä 9.32 (d, J) 7.2 Hz, 3H), 7.90 (d,J) 7.2 Hz, 3H), 7.72 (t, J) 7.2 Hz, 3H), 7.60 (d, J) 7.2 Hz,3H).

Second step: Synthesis of 2,2',2"-(5H-diindeno[1,2-a:1',2'-c]fluorene-5,10,15-triylidene)trimalononitrile (2). All manipulations were carried out in air.

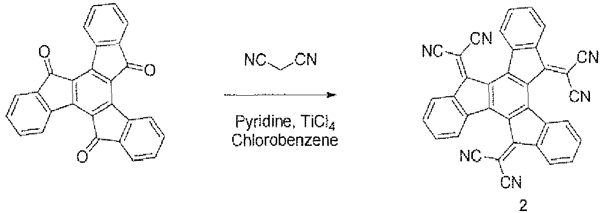

1 mmol of 1 was dissolved in 50 ml of dry deoxygenated chlorobenzene. Then 6 mmol of malononitrile was added under Argon followed by a dropwise addition of 6 mmol of TiCl₄ and 12 mmol of dry pyridine during 2h under vigorous stirring. The reaction mixture was warmed to 70 °C and stirred for another 4h. Water (30 ml) was added to the reaction mixture and the product was extracted with methylene chloride. Column chromatography (methylene chloride as the eluent) afforded 2. The residue was recrystallized from chlorobenzene to afford C-2 as red crystals (m.p.> 250 °C) in 42% yield.

This second step is very versatile and can be used in order to obtain materials: C-3-11, C18 by using the appropriate C-H acidic compound of structure CH-3-11 respectively:

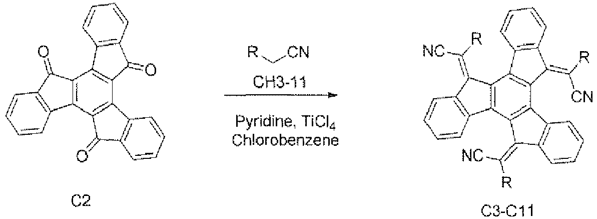

| CH-3 | CH-4 | CH-5 | CH-6 |
|---|---|---|---|
| 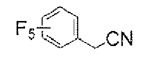 | 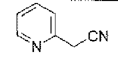 | 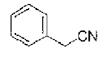 | 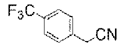 |

| CH-7 | CH-8 | CH-9 | CH-10 |
|---|---|---|---|
| 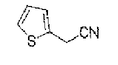 | 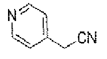 | 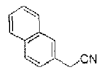 | 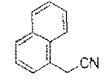 |

| CH-11 | | | |
|---|---|---|---|
| 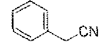 | | | |

The synthetic procedure can be used as such to obtain each of C3-11 in order to obtain them in sufficient purity and quantity. Some slight alteration of the washing or purification steps may occur in some occasions. Someone skilled in the art will be aware that in the preparation of some compounds some slight alterations of the washing or purification steps might be necessary without changing the general principle of the preparation method.

Compound C-2 has a very high conductivity in a doped form, if compared to other organic ETMs. The conductivity at room temperature is 1,06·10⁻³ S/cm and the stability temperature is 153 °C for a layer doped with Tetrakis(1,3,4,6,7,8-Hexahydro-2H-pyrimido[1,2-a]pyrimidinato)ditungsten (II). The conductivity at room temperature is 1,2·10⁻² S/cm and the stability temperature is 141 °C for a layer doped with 4,4',5,5'-tetracyclohexyl-1,1',2,2',3,3'-hexamethyl-2,2',3,3'-tetrahydro-1H,1'H-2,2'-biimidazole.

### Example C-21:

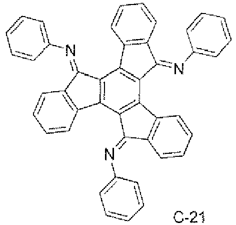

### Step 1:

First step: Synthesis of 5H-diindeno[1,2-a:1',2'-c]fluorene-5,10,15-trione (1). All manipulations were carried out in air, without any further purification of commercial solvents/chemicals.

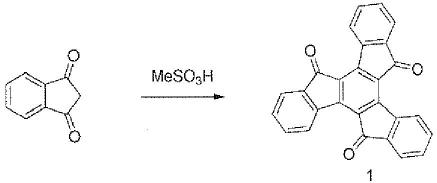

Truxenone. In a 100 mL round-bottom flask with a magnetic stir bar was added indan-1,3-dione (2.50 g, 17.0 mmol) and methaneesulfonic acid (40 mL). The mixture was heated at 110 °C for 3 h. After being cooled to room temperature, the reaction mixture was dispersed in water (300 mL) and the crude product was filtered off. The product was dissolved in hot propylene carbonate (75 mL) and after being cooled was isolated by suction filtration.
Yield: 1.73 g (79%).
1H NMR (CDCl3): ä 9.32 (d, J) 7.2 Hz, 3H), 7.90 (d,J)7.2 Hz, 3H), 7.72 (t, J) 7.2 Hz, 3H), 7.60 (d, J) 7.2 Hz,3H).

### Second step:

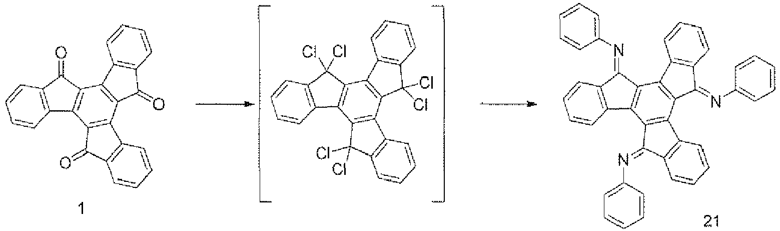

2,5mmol of 1 is dissolved in 90ml thienylchloride and 1 mL THF (cat quantity) and refluxed 3 days under an argon atmosphere. Thienylchloride is then distilled off and the residue was kept under argon (5,5,10,10,15,15-hexachloro-10,15-dihydro-5H-diindeno[1,2-a:1',2'-c]fluorene). In another flask 15mmol aniline is dissolved in 10mL glyme and poured at -10°C into a third flask previously charged with 60% sodium hydride (1,26g 31,5mmol) and 200ml Glyme, the mixture is stirred at -10°C for half an hour and 1.5 hour at Room Temperature during which time a gas evolution can be noticed. After this time, the solution is cooled down to -78°C. To this solution was added The 5,5,10,10,15,15-hexachloro-10,15-dihydro-5H-diindeno[1,2-a:1',2'-c]fluorene which was then dissolved in 15 mL dry dichlotomethane, under argon. This mixture is then stirred during 3 days at room temperature, under argon before being heated up to 50°C for 24 hours.

### Work up:

The mixture was then cooled down to room temperature, poured onto 500mL ice water, and extracted with dichloromethane. The solvent was then removed by rotary evaporation. The obtained oil was then treated with diethylether (100mL). The suspension was filtered off, and gel filtrated (Dichloromethane) over a silica pad, to obtain an orange solid after dichloromethane is removed.
Yield: 35mg (2%), 97% HPLC purity.
Cyclovoltammetry: -0,89 V vs Fc.

### Device examples

Device 1 (comparative). A state of the art organic solar cell can be fabricated with the following procedure: patterned glass substrate coated with ITO is cleaned in an ultrasound bath with ethanol, acetone and isopropanol. Afterwards the ITO substrate is exposed to oxygen plasma treatment for 15 minutes. The substrate is loaded into the vacuum trough a glove box with nitrogen. In vacuum the organic layers are deposited with conventional VTE (vacuum thermal evaporation). First a 10 nm thick 5% (molar) p-doped CuPc layer is deposited through a shadow mask over the ITO. A 10 nm undoped CuPc layer is deposited over the doped CuPc layer. A 30 nm thick mixed layer of fullerene C60 and CuPc is deposited with a molar ratio of 2(C60):1(CuPc). A 40 nm thick C60 layer is deposited on top of the mixed layer. A 10 nm BPhen (4,7-diphyenyl-1,10-phenanthroline) layer is deposited on top of the C60 layer. The BPhen layer is followed by a 100 nm thick Al cathode. Under standard simulated AM1.5 (Air Mass 1.5) solar spectra, such a device typically shows a short circuit current of about 8 mA/cm², a FF of about 40 % and an open circuit voltage of about 0.5 V.

Device 2. An inventive organic solar cell can be made with the same layer structure as device 1 except that a 10 nm thick n-doped layer of the compound C-2 is used instead of the BPhen layer. Under standard simulated AM1.5 such a device typically shows increased performance with a short circuit current of 8 mA/cm², a FF of 45 % and an open circuit voltage of 0.53 V. The short circuit can be further improved by replacing part of the 40 nm thick C60 layer with the compound C-2, and optimizing the optical cavity of the device.

On a thermal stress test, a solar cell according to device 1 will stop to work at 67 °C whereas a device 2 can work under temperatures of at least 80 °C.

### Nomenclature

Inverted - The term inverted solar cell, or inverted structure, refers to a device with a layer structure in which the cathode is closer to the substrate than the anode. In the method of production of an inverted device, the cathode is formed on the substrate, following the deposition of the organic and other layers, which are followed by the deposition of the cathode.

ETL - electron transport layer, is a layer which is used in a device stack in such a way that the main charge carriers are electrons. Typically, this layer comprises an electron transport material (ETM). Hole blocking layers, exciton blocking layers between the cathode and its closest donor-acceptor heterojunction are also electron transport layers. Electron injection layers could also be electron transport layers, if they are semiconductors comprising an ETM.

ETM - electron transport material is a semiconducting material which is stable towards reduction and has a high mobility for electrons. In an ETM, the electron mobility is typically higher than the hole mobility.

HTL - hole transport layer, is a layer which is used in a device stack in such a way that the main charge carriers are electrons. Typically this layer comprises a hole transport material (HTM).

HTM - hole transport material is a semiconducting material which is stable towards oxidation and has a high mobility for holes. In a HTM, the hole mobility is typically higher than the electron mobility.

FHJ - Flat heterojunction, is a donor-acceptor heterojunction in which the donor and acceptor materials are in separate layers. Preferentially the donor and acceptor materials are in adjacent layers providing a hetero-interface. Alternatively, other layers can be placed in between, to assist the light absorption and/or charge carrier separation.

BHJ - Bulk heterojunction, is a mixed layer comprising a donor, an acceptor, and an absorbing material. Typically at least one of the donor and acceptor materials are also the absorbing material. The donor-acceptor heterointerface is necessary for the separation of the excitons formed by photoabsorbtion into charge carriers. A bulk heterojunction can be graded, or also comprise additional layers. A bulk donor-acceptor heterojunction can also be a hybrid junction, comprising a mixed layer and at least one layer comprising: the acceptor but no donor material, or the donor but no acceptor material. Such a heterojunction can also be a graded bulk heterojunction.

Acceptor - Acceptor, in this invention, is a compound used in an optically active layer of a solar cell to assist the excitonic separation into charge carriers, accepting the electron. The term acceptor must not be confused with an electrical p-dopant which is a very strong acceptor capable of doping a hole transport layer.

Donor - Donor, in this invention, is a compound used in an optically active layer of a solar cell to assist the excitonic separation into charge carriers, donating an electron (accepting a hole). The term donor must not be confused with an electrical n-dopant which is a very strong donor capable of doping an electron transport layer.

Electrical dopant - Electrical dopant is a dopant which is capable to, when added to a semiconductor, increase its charge carrier density, consequently increasing its conductivity. The increase in charge carrier density is due to a charge transfer between the LUMO and HOMO of the at least two components of the dopant-semiconductor system. The term electrically doped refers to a layer or material which is doped by an electrical dopant, as defined above.

n-dopant - electrical dopant capable of increasing the density of negative charge carriers in an electron transport material or electron transport layer. The negative charge carriers are provided on the effective conduction band of the electron transport layer (typically the LUMO of the electron transport material).

p-dopant - electrical dopant capable of increasing the density of positive charge carriers in a hole transport material or hole transport layer. The positive charge carriers are provided on the effective valence band of the hole transport layer (typically the HOMO of the hole transport material).

Transparency - those transport layers, which do not contribute to the photocurrent generation, are required to be transparent to avoid any efficiency loss due to undesired absorption. A high transparency is required in the range of wavelengths in which the solar cell is active. A high transparency preferentially means an extinction coefficient (k) smaller than 1, more preferably smaller than 0.1.

LUMO - Lowest unoccupied molecular orbital.

HOMO - Highest occupied molecular orbital.

Intrinsic layer - a layer which is not doped with dopants which increases the charge carrier density in the layer. Here it is considered that the layer in the dark, and no temperature gradient, or electrical field is applied to it.

The features disclosed in the foregoing description and in the claims may, both separately and in any combination thereof, be material for realizing the invention in diverse forms thereof.

## Claims

1. An organic solar cell comprising at least one compound according to the following formula (I): 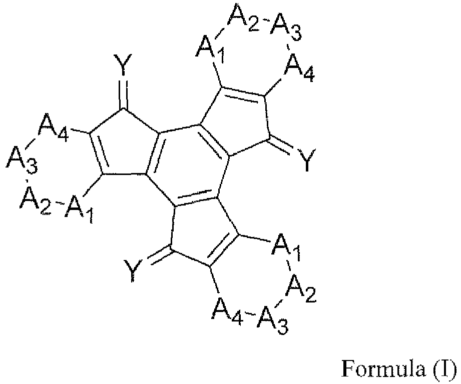 wherein
Y is selected from N-R₁ or R₁-C-R₂;
R₁ and R₂ are independently selected from CN, aryl, alkyl, NO₂, halogen, heteroaryl, CF₃; aryl can be substituted to form perfluorophenyl and/or fluorophenyl;
A₁,A₂,A₃,A₄ are independently selected from C, CH, N, CR and form an aromatic ring, or at least one of A₁-A₂, A₂-A₃, A₃-A₄ is part of an additional condensed aromatic ring;
R is an aromatic ring
or
the compound according to formula (I) is selected from a compound according to the following formulae (Ia) and (Ib) 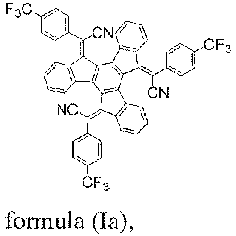 or

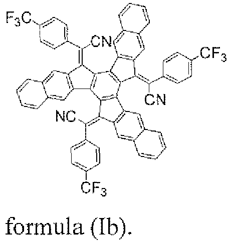

2. The organic solar cell according to claim 1, comprising at least one first electron transport layer, which electron transport layer comprises a material according to Formula (I) of claim 1.

3. The organic solar cell according to claim 2, wherein the electron transport layer is doped.

4. The organic solar cell according to claim 2 or 3, comprising an additional electron transport layer wherein the first electron transport layer is doped and the additional electron transport layer is undoped, and wherein the additional electron transport layer is adjacent to a donor-acceptor heterojunction and the first electron transport layer is in between the additional electron layer and a cathode.

5. The organic solar cell according to claim 4, wherein the additional electron transport layer is at least one of hole blocking layer and exciton blocking layer.

6. The organic solar cell according to any of the previous claims, wherein a donor-acceptor heterojunction comprises a material according to Formula (I) of claim 1, preferentially as acceptor.

7. The organic solar cell according to claim 6, wherein the donor-acceptor heterojunction is a bulk-heterojunction.

8. The organic solar cell according to claim 1, comprising at least one semiconductor polymer layer and one electron transport layer, which electron transport layer comprises a material according to Formula (I) of claim 1.

9. The organic solar cell according to claim 8, wherein the electron transport layer is n-doped.

10. Compound

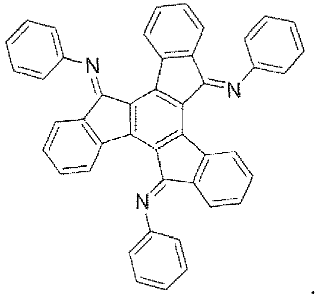

## Patentansprüche

1. Organische Solarzelle, umfassend zumindest eine Verbindung gemäß der folgenden Formel (I): 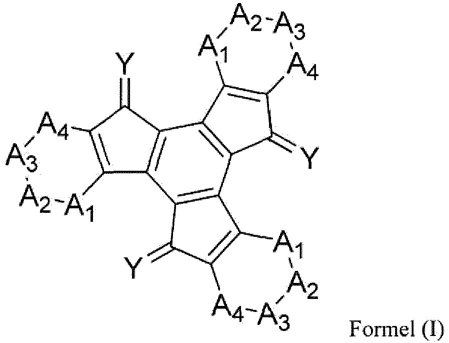 wobei
Y ausgewählt ist aus N-R₁ oder R₁-C-R₂;
R₁ und R₂ unabhängig voneinander ausgewählt sind aus CN, Aryl, Alkyl, NO₂, Halogen, Heteroaryl, CF₃; wobei das Aryl substituiert sein kann, um Perfluorophenyl und/oder Fluorophenyl zu bilden;
A₁, A₂, A₃, A₄ unabhängig voneinander ausgewählt sind aus C, CH, N, CR und einen aromatischen Ring bilden, oder zumindest eines von A₁-A₂, A₂-A₃, A₃-A₄ Teil eines zusätzlichen kondensierten aromatischen Rings ist;
R eine aromatischer Ring ist
oder
die Verbindung gemäß der Formel (I) ausgewählt ist aus einer Verbindung gemäß den folgenden Formeln (Ia) und (Ib) 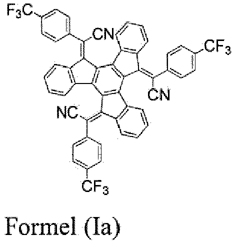 oder

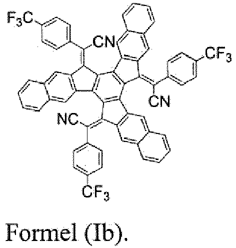

2. Organische Solarzelle nach Anspruch 1, umfassend zumindest eine erste Elektronentransportschicht, wobei die Elektronentransportschicht ein Material gemäß der Formel (I) des Anspruch 1 umfasst.

3. Organische Solarzelle nach Anspruch 2, wobei die Elektronentransportschicht dotiert ist.

4. Organische Solarzelle nach Anspruch 2 oder 3, umfassend eine zusätzliche Elektronentransportschicht, wobei die erste Elektronentransportschicht dotiert ist und die zusätzliche Elektronentransportschicht undotiert ist und wobei die zusätzliche Elektronentransportschicht zu einem Donor-Akzeptor-Heteroübergang benachbart ist und die erste Elektronentransportschicht zwischen der zusätzlichen Elektronentransportschicht und einer Kathode ist.

5. Organische Solarzelle nach Anspruch 4, wobei die zusätzliche Elektronentransportschicht zumindest eine einer Loch-blockenden Schicht und einer Excitonen-blockenden Schicht ist.

6. Organische Solarzelle nach einem der vorangehenden Ansprüche, wobei eine Donor-Akzeptor-Heteroverbindung zumindest ein Material gemäß der Formel (I) des Anspruch 1 umfasst, vorzugsweise als Akzeptor.

7. Organische Solarzelle nach Anspruch 6, wobei die Donor-Akzeptor-Heteroverbindung eine Festkörper(bulk)-Heteroverbindung ist.

8. Organische Solarzelle nach Anspruch 1, umfassend zumindest eine halbleitende Polymerschicht und eine Elektronentransportschicht, wobei die Elektronentransportschicht ein Material gemäß der Formel (I) des Anspruch 1 umfasst.

9. Organische Solarzelle nach Anspruch 8, wobei die Elektronentransportschicht n-dotiert ist.

10. Verbindung

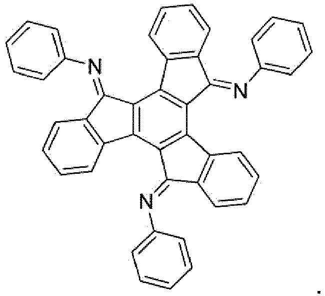

## Revendications

1. Cellule photovoltaïque organique comprenant au moins un composé selon la formule (I) suivante : 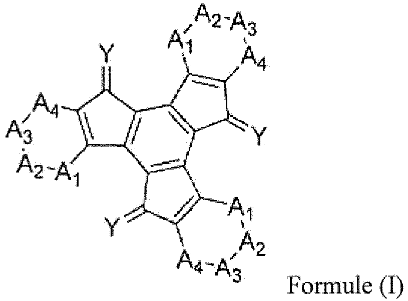 dans laquelle
Y est choisi parmi N-R₁ ou R₁-R₂ ;
R₁ et R₂ sont indépendamment choisis parmi CN, l'aryle, l'alkyle, NO₂, l'halogène, l'hétéroaryle, CF₃ ;
l'aryle peut être substitué pour former du perfluorophényle et/ou du fluorophényle ;
A₁, A₂, A₃, A₄ sont indépendamment choisis parmi C, CH, N, CR et forment un noyau aromatique, ou au moins un de A₁-A₂, A₂-A₃, A₃-A₄ fait partie d'un noyau aromatique condensé supplémentaire ;
R est un noyau aromatique
ou
le composé selon la formule (I) est choisi parmi un composé selon les formules (Ia) et (Ib) suivantes : 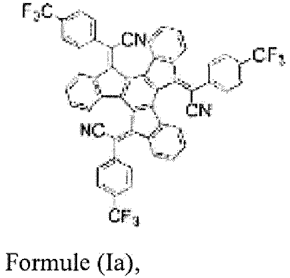 ou

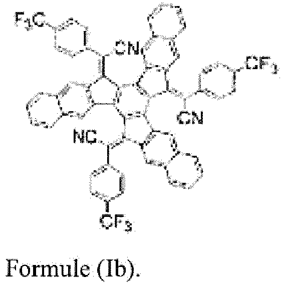

2. Cellule photovoltaïque organique selon la revendication 1, comprenant au moins une première couche de transport d'électrons, laquelle couche de transport d'électrons comprend un matériau selon la Formule (I) de la revendication 1.

3. Cellule photovoltaïque organique selon la revendication 2, dans laquelle la couche de transport d'électrons est dopée.

4. Cellule photovoltaïque organique selon la revendication 2 ou 3, comprenant une couche de transport d'électrons supplémentaire, dans laquelle la couche de transport d'électrons est dopée et dans laquelle la couche de transport d'électrons supplémentaire n'est pas dopée, et dans laquelle la couche de transport d'électrons supplémentaire est adjacente à une hétérojonction donneur-accepteur et la première couche de transport d'électrons se situe entre la couche d'électrons supplémentaire et une cathode.

5. Cellule photovoltaïque organique selon la revendication 4, dans laquelle la couche de transport d'électrons supplémentaire est au moins l'une d'une couche de blocage des trous et d'une couche de blocage des excitons.

6. Cellule photovoltaïque organique selon l'une quelconque des revendications précédentes, dans laquelle une hétérojonction donneur-accepteur comprend un matériau selon la Formule (I) de la revendication 1, de préférence en tant qu'accepteur.

7. Cellule photovoltaïque organique selon la revendication 6, dans laquelle l'hétérojonction donneur-accepteur est une hétérojonction en vrac.

8. Cellule photovoltaïque organique selon la revendication 1, comprenant au moins une couche de polymères semi-conducteurs et une couche de transport d'électrons, laquelle couche de transport d'électrons comprend un matériau selon la Formule (I) de la revendication 1.

9. Cellule photovoltaïque organique selon la revendication 8, dans laquelle la couche de transport d'électrons est dopée n.

10. Composé
